# EUROPEAN PATENT APPLICATION

(11) **EP 3 132 777 A1**
(43) Date of publication of application: **22.02.2017**
(21) Application number: 15779613.7
(22) Date of filing: 16.04.2015
(51) Int. Cl.: A61F 11/08, A61F 11/10

(54) **EARPLUGS AND EARPLUG SET HAVING SAME**

(30) Priority: 17.04.2014 KR 20140045765
(71) Applicant: Chae, Seung Ho, Paju-si, Gyeonggi-do 413-110 (KR)
(72) Inventor: CHAE, Seung Ho, Paju-si, Gyeonggi-do 413-110 (KR)
(74) Representative: Ruzzu, Giammario
(86) International application number: PCT/KR2015/003824
(87) International publication number: WO 2015/160196

(57) **Abstract**

Disclosed are earplugs capable of providing a good feel when worn, buffering external impact, and blocking noise or controlling the volume of the noise coming in from the outside. The earplugs comprise: a main body having a small cross-sectional part and an extended part; and an ear pad, which is mounted on the outer circumferential surface of the small cross-sectional part, comes into contact with the inner circumferential surface of an ear canal when worn in an ear, and blocks the gap between the outer circumferential surface of the small cross-sectional part and the inner circumferential surface of the ear canal, the earplugs also comprising a flexible part, which is installed between the small cross-sectional part and the extended part and allows the adjustment of the position of the extended part with respect to the small cross-sectional part.

## Description

### [Technical Field]

The present invention relates to an earplug and an earplug set having the same, and more particularly, to an earplug and an earplug set having the same which are used to block noise or loud sounds and to protect the eardrum from loud sounds or noise.

### [Background Art]

In general, when workers work at a work site where a very bad sound is generated, when persons get into a helicopter or when persons who are sensitive to noise want to get out of noise, they wear earplugs to block a sound or noise or to control the volume of a sound or noise.

Korean Patent Publication No. 10-2005-0033009 discloses an earplug with an advertising cap which has been invented by Park Jin Yong. In Korean Patent Publication No. 10-2005-0033009, the earplug includes: a main body; an earhole insertion part which is formed integrally with one side of the main body to be inserted into the earhole; a filter which is inserted into a through hole formed inside the main body; a disc-shaped advertising cap having an advertising surface formed on one side and an insertion part formed on the other side; and an insertion recess which is formed in the opposite side of the earhole insertion part of the main body so that the insertion part of the advertising cap is inserted into the insertion recess, thereby protecting a user's hearing due to sound insulation of the earplug and obtaining advertising effect and publicity effect at the same time.

Such a conventional earplug has a disadvantage in that users may feel discomfort according to physical structures of the users' ears because it is difficult to transform the main body of the earplug. In order to overcome such a disadvantage, main bodies of various shapes according to the physical structures of user's ears must be made.

Moreover, the conventional earplug has further several disadvantages in that there is a concern that the user may be injured because external impact applied to the main body is transferred to the user's ear as it is, and in that it is inconvenient to use because the user has to take out the earplug from the ear when the user wants to talk with someone and wear the earplug again after talking.

Furthermore, the conventional earplug has another disadvantage in that it is difficult to use it at places where it is necessary to talk with someone or hear specific sounds while controlling the volume of noise because it is difficult to hear outside sounds when the user wears the earplug.

### [Disclosure]

### [Technical Problem]

Accordingly, the present invention has been made in an effort to solve the above-mentioned problems occurring in the prior arts, and it is an object of the present invention to provide an earplug which can be transformed adaptationally according to physical structures of users' ears so that many people can use the earplugs conveniently compared with the conventional earplugs.

It is another object of the present invention to provide an earplug which can buffer external impact applied to a main body.

It is a further object of the present invention to provide an earplug which can remarkably reduce outside noise and allow the user to hear necessary sounds.

It is a still further object of the present invention to provide an earplug which can control the volume of sounds from the outside.

It is another object of the present invention to provide an earplug which can provide convenience in keeping the earplug in safety and in distributing the earplugs.

### [Technical Solution]

To achieve the above objects, the present invention provides an earplug including: a main body having a small cross-sectional part and an extended part; an ear pad which is mounted on the outer circumferential surface of the small cross-sectional part and comes into contact with the inner circumferential surface of an ear canal when a user wears the earplug in the ear, so as to block a gap between the outer circumferential surface of the small cross-sectional part and the inner circumferential surface of the ear canal; and a flexible part which is mounted between the small cross-sectional part and the extended part and allows the adjustment of the position of the extended part with respect to the small cross-sectional part.

Preferably, the flexible part includes a corrugated part which can buffer external impact. Preferably, the main body includes a volume controlling hole formed along the inside of the main body to penetrate through the small cross-sectional part, the flexible part and the extended part, and the extended part includes volume controlling means mounted to block the volume controlling hole from the outside or to open the volume controlling hole to the outside.

Preferably, the volume controlling means includes: a support frame having a rotation support part, which is formed on the outer surface thereof and is mounted at an opening formed at an end portion of the extended part to block the opening, and one or more first through holes, which are formed at one or more portions to communicate the inner space of the extended part with the outside; and a rotating member having a hand-grip for adjusting a rotational angle and one or more second through holes, which are formed to make the rotation support member rotatable and are arranged to be overlapped with the first through hole at one or more portions according to the rotational angle to communicate the inner space of the extended part with the outside or to block the first through hole by releasing the state that the first through hole and the second through hole are overlapped with each other.

Preferably, the support frame includes a filter mounting part formed on the inner surface thereof and a moisture-proofing filter for removing howling and preventing moisture is mounted on the filter mounting part.

Preferably, a plurality of the first through holes and a plurality of the second through holes are formed, and the number of overlaps of the first through holes and the second through holes is adjustable to be in plural.

The present invention provides an earplug set includes: a pair of earplugs; a lower case including earplug accommodating parts for accommodating a pair of the earplugs and a pair of ear pad mounting parts, each of which has a protrusion part formed at the center to allow the user to stick ear pads thereto; at least a pair of ear pads mounted on a pair of the ear pad mounting parts; and an upper case which is opposed to the lower case and surrounds a pair of the earplugs and a pair of the ear pads to protect them.

### [Advantageous Effects]

The earplug and the earplug set having the same according to the present invention can provide a good feeling of wearing for persons who have various physical structures of ears because the flexible part for adjusting the position of the extended part with respect to the small cross-sectional part is disposed between the small cross-sectional part and the extended part of the main body.

The earplug and the earplug set having the same according to the present invention can lower possibility that the user gets hurt because the corrugated part of the flexible part buffers external impact applied to the main body.

The earplug and the earplug set having the same according to the present invention can control the volume of sounds from the outside while the user is wearing the earplug, and allow the user to talk with others or hear necessary sounds while wearing the earplug as occasion demands.

The earplug and the earplug set having the same according to the present invention can block or reduce soundwaves introduced from the outside in the state where the user is wearing the earplug.

The earplug and the earplug set having the same according to the present invention can remove howling doubly by the corrugated part and the moisture-proofing filter.

### [Description of Drawings]

FIG. 1 is a perspective view of an earplug according to a preferred embodiment of the present invention.
FIG. 2 is an exploded perspective view of the earplug of FIG. 1.
FIG. 3 is a sectional view taken along the line of I-I of FIG. 1.
FIG. 4 is a sectional view taken along the line of J-J of FIG. 1.
FIG. 5 is a view showing a state that the volume is controlled by a first through hole formed in a support frame and a second through hole formed in a rotating member.
FIG. 6 is a sectional view showing an earplug set having the earplug according to the preferred embodiment of the present invention.
FIG. 7 is a sectional view of a lower case of FIG. 6.
FIG. 8 is a sectional view of an upper case of FIG. 6.

### [Mode for Invention]

Hereinafter, reference will be now made in detail to the preferred embodiment of the present invention with reference to the attached drawings.

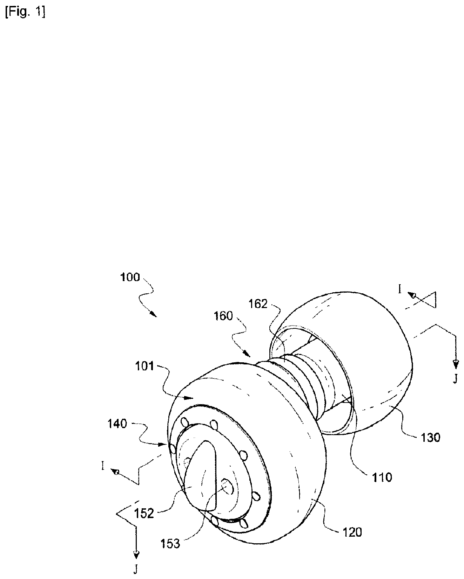

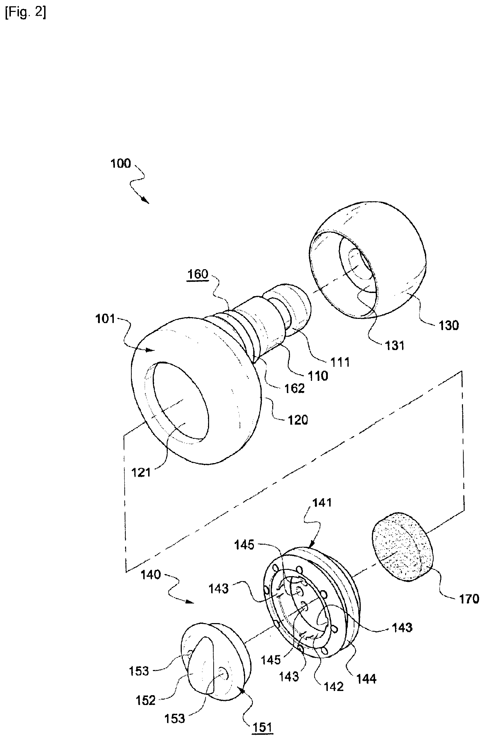

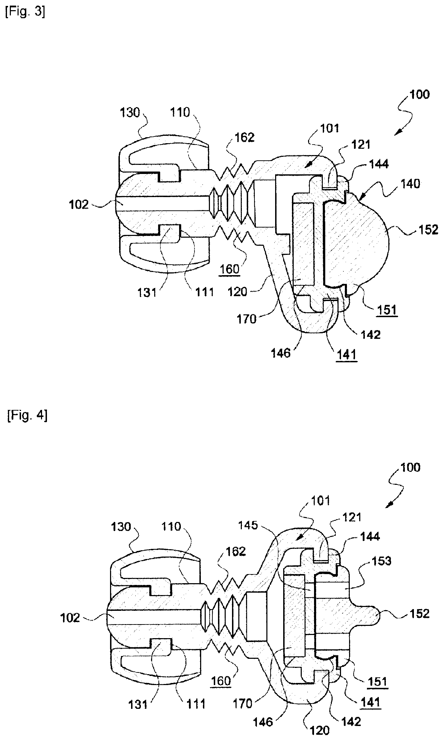

FIG. 1 is a perspective view of an earplug according to a preferred embodiment of the present invention, FIG. 2 is an exploded perspective view of the earplug of FIG. 1, FIG. 3 is a sectional view taken along the line of I-I of FIG. 1, and FIG. 4 is a sectional view taken along the line of J-J of FIG. 1.

As shown in FIGS. 1 to 4, the earplug 100 according to the preferred embodiment of the present invention includes: a main body 101 having a small cross-sectional part 110 and an extended part 120; an ear pad 130 combined to the outer circumferential surface of the small cross-sectional part 110 of the main body 101; and volume controlling means 140 mounted on the extended part 120 of the main body 101.

The main body 101 has a volume controlling hole 102 penetrating through the small cross-sectional part 110 and the expended part 120, and the small cross-sectional part 110 has a groove 111 for preventing separation of the ear pad 130 mounted.

The extended part 120 has an opening 121 which is formed in a cross-sectional part thereof to communicate the volume controlling hole 102 with the outside.

A flexible part 160 is formed between the small cross-sectional part 110 and the extended part 120 of the main body 101. The flexible part 160 can adaptationally adjust the position of the extended part 120 with respect to the small cross-sectional part 110 according to physical structures of users' ears so as to provide a comfortable feeling of wearing. The volume controlling hole 102 penetrates through the flexible part 160. As shown in the drawing, the flexible part 160 has a corrugated part 162 in order to buffer external impact applied to the main body 101. It is appropriate to form three corrugations on the corrugated part 162, but the number of corrugations may be increased or decreased. If the corrugated part 162 is formed on the flexible part 160, it may provide refraction effect of sound waves passing through the inside of the main body and removal effect of howling. It is preferable that the main body 101 be made of urethane.

The ear pad 130 is mounted on the outer circumferential surface of the small cross-sectional part 110, is formed in a cylinder with a double-wall, and has a combining jaw 131 which protrudes inwardly at the inner end portion of the inner circumferential surface and is combined to the groove 111. When the user wears the earplug in the ear, the ear pad 130 comes into contact with the inner circumferential surface of an ear canal to block a gap between the outer circumferential surface of the small cross-sectional part 110 and the inner circumferential surface of the ear canal. The ear pad 130 is fixed to the ear canal because having self-elasticity. Preferably, such an ear pad 130 is made of silicon. The volume controlling means 140 is mounted on the extended part 120 through the opening 121. The volume controlling means 140 blocks the volume controlling hole 102 from the outside or makes a passage of sound waves getting from the outside large or small by opening the volume controlling hole 102 so as to control the volume of sounds that the user can hear.

The volume controlling means 140 includes a support frame 141 and a rotating member 151. The support frame 141 has a rotation support part 142 formed on the outer surface thereof and is mounted at the opening 121 formed at an end portion of the extended part 120 to block the opening 121. The inner circumferential surface of the rotation support part 142 has an outwardly projecting form to prevent the rotating member 151 combined to the rotation support part from being easily separated. Preferably, the volume controlling means 140 has split recesses 143 which are inwardly formed on the outer rim of the rotation support part 142 to be spaced apart from one another at several intervals so as to forcedly assemble or disassemble the rotating members 151 when a certain power is applied.

A ring-shaped groove 144 is formed along the outer circumferential surface of the support frame 141 to be fixed to the opening 121. One or more first through holes 145 are formed on one or more portions of the support frame 141 in order to communicate the inner space of the extended part 120 with the outside. Preferably, at least two or more first through holes 145 are formed. In this embodiment, three through holes 145 are mounted on the half of the circumference within the same radius at several intervals, and one through hole 145 is mounted on the opposite half of the circumference, and so, total four through holes 145 are formed. As occasion demands, two first through holes 145 may be formed at symmetrical locations one by one based on the center. Moreover, as occasion demands, just one round through hole or an elongated hole may be formed. Namely, the through holes may be formed in various shapes and areas.

Preferably, a filter mounting part 146 is formed on the inner surface of the support frame 141, and a moisture-proofing filter 170 for removing howling and for moisture-proofing is mounted on the filter mounting part 146. The moisture-proofing filter 170 is preferably made of PS-foamed material. In this embodiment, the moisture-proofing filter 170 is mounted on the filter mounting part 146 of the support frame 141, but may be mounted and fixed on the main body 101 inside the extended part 120, inside the volume controlling hole 102 or other parts.

Furthermore, the support frame 141 has scales or grades indicated on the outer surface thereof so that the user can easily match a hand-grip 152 at a wanted position.

The rotating member 151, preferably, has the hand-grip 152 protruding toward the outer surface. The hand-grip 152 is to adjust a rotational angle of the rotating member 151.

The rotating member 151 is mounted on the rotation support part 142 to be able to rotate. Because the outer circumferential surface of the rotating member 151 adopts the outwardly projecting form, the rotating member 151 does not get out of the rotation support part 142 unless a great external force is applied.

The rotating member 151 has a second through hole 153. A plurality of the second through hole 153 may be formed, but as occasion demands, just one second through hole 153 may be also formed. The second through hole 153 may be formed in various shapes and areas. In this embodiment, two second through holes 153 are formed at both sides one by one based on the hand-grip 152

Preferably, the support frame 141 and the rotating member 151 are made of ABS resin, but may be made of other plastic materials.

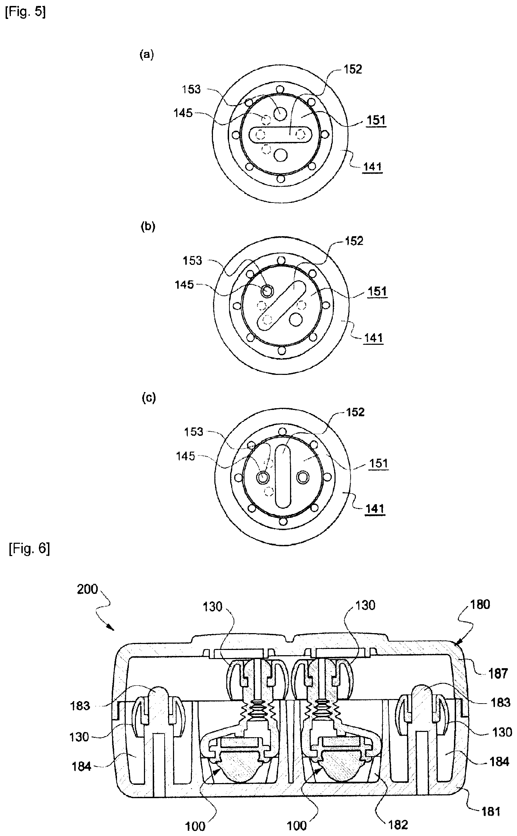

FIG. 5 is a view showing a state that the volume is controlled by the first through holes formed in the support frame and the second through holes formed in the rotating member.

FIG. 5(a) illustrates the state that the second through holes 153 are not overlapped with the first through holes 145 (zero stage), FIG. 5(b) illustrates the state that one of the second through holes 153 and one of the first through holes 145 are overlapped with each other (one stage), and FIG. 5(c) illustrates the state that two of the second through holes 153 and two of the first through holes 145 are overlapped with each other (two stages) in order to control the volume.

In other words, the earplug 100 according to the present invention can block outside noise or sounds when the user grasps the hand-grip 152 and rotates the rotating member 151 as shown in FIG. 5(a) while wearing the earplug 100 in the ear, turn down noise or sounds when one of the first through holes 145 and one of the second through holes 153 are overlapped with each other as shown in FIG. 5(b), or control the volume of noise or sounds to be increased more than the sounds of FIG. 5(b) when two of the first through holes 145 and two of the second through holes 153 are overlapped with each other as shown in FIG. 5(c).

### [Best Mode]

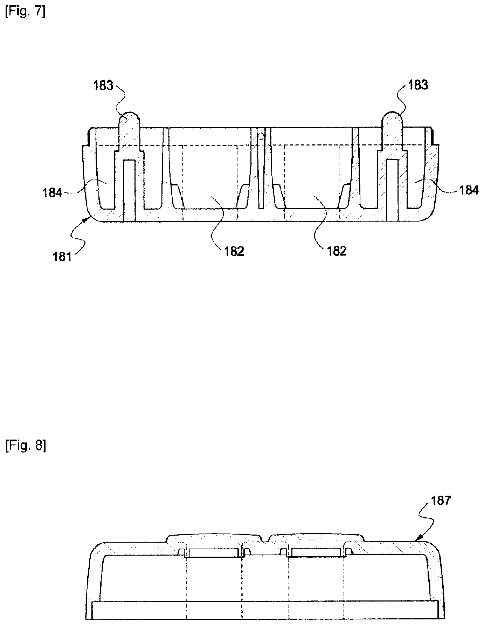

FIG. 6 is a sectional view showing an earplug set having the earplug according to a preferred embodiment of the present invention, FIG. 7 is a sectional view of a lower case of FIG. 6, and FIG. 8 is a sectional view of an upper case of FIG. 6.

As shown in FIGS. 6 to 8, the earplug set 200 according to the preferred embodiment of the present invention includes a pair of earplugs 100 described with reference to FIGS. 1 to 4 and a case 180 which accommodates a pair of the earplugs 100 therein to protect the earplugs 100.

A pair of the earplugs 100 are the same as the earplugs 100 described above.

The case 180 includes: a lower case 181; and an upper case 187 which is opposed to the lower case 181 and is combined to surround the earplug 100. As shown in FIGS. 6 and 7, the lower case 181 includes: earplug accommodating parts 182 for accommodating a pair of the earplugs 100; and a pair of ear pad mounting parts 184 each of which has a protrusion part 183 formed at the center to allow the user to fix the ear pad 130 thereto.

Preferably, ear pads which are different in size from the ear pads 130 mounted on the earplugs 100 are fixed to the protrusion part 183 so that the user can select ear pads which fit to the user's ears.

As shown in FIGS. 6 and 8, the upper case 187 is opposed to the lower case 181 and surrounds a pair of the earplugs 100 and a pair of the ear pads 130 to protect them and prevent foreign matters from getting into the case 180.

The upper case 187 and the lower case 181 are all made of styrene acrylonitrile copolymers (SAN) with excellent transparency, thermos-mechanical force, chemical resistance and glossiness.

As described above, when the earplug 100 according to the present invention is put in the case 180 having the lower case 181 and the upper case 187, the earplug 100 can be easily stored and carried in safety and can be protected from dust or foreign matters.

Additionally, because the ear pads 130 which are different in size from the ear pads 130 mounted on the earplugs 100 are put in the ear pad mounting parts 184 of the case 180, the user can select ear pads which fit to the user's ears.

### [Industrial Applicability]

The present invention is usable in the field of manufacturing earplugs and an earplug set which can control the level of noise or outside sounds, buffer external impact and remove howling.

## Claims

1. An earplug which includes: a main body having a small cross-sectional part and an extended part; and an ear pad which is mounted on the outer circumferential surface of the small cross-sectional part and comes into contact with the inner circumferential surface of an ear canal when a user wears the earplug in the ear, so as to block a gap between the outer circumferential surface of the small cross-sectional part and the inner circumferential surface of the ear canal, the earplug comprising:
a flexible part which is mounted between the small cross-sectional part and the extended part and allows the adjustment of the position of the extended part with respect to the small cross-sectional part.

2. The earplug according to claim 1, wherein the flexible part includes a corrugated part which can buffer external impact.

3. The earplug according to claim 1, wherein the main body includes a volume controlling hole formed along the inside of the main body to penetrate through the small cross-sectional part, the flexible part and the extended part, and the extended part includes volume controlling means mounted to block the volume controlling hole from the outside or to open the volume controlling hole to the outside.

4. The earplug according to claim 2, wherein the main body includes a volume controlling hole formed along the inside of the main body to penetrate through the small cross-sectional part, the flexible part and the extended part, and the extended part includes volume controlling means mounted to block the volume controlling hole from the outside or to open the volume controlling hole to the outside.

5. The earplug according to claim 3 or 4, wherein the volume controlling means includes: a support frame having a rotation support part, which is formed on the outer surface thereof and is mounted at an opening formed at an end portion of the extended part to block the opening, and one or more first through holes, which are formed at one or more portions to communicate the inner space of the extended part with the outside; and a rotating member having a hand-grip for adjusting a rotational angle and one or more second through holes, which are formed to make the rotation support member rotatable and are arranged to be overlapped with the first through hole at one or more portions according to the rotational angle to communicate the inner space of the extended part with the outside or to block the first through hole by releasing the state that the first through hole and the second through hole are overlapped with each other.

6. The earplug according to claim 5, wherein the support frame includes a filter mounting part formed on the inner surface thereof and a moisture-proofing filter for removing howling and preventing moisture is mounted on the filter mounting part.

7. The earplug according to claim 5, wherein a plurality of the first through holes and a plurality of the second through holes are formed, and the number of overlaps of the first through holes and the second through holes is adjustable to be in plural.

8. An earplug set having the earplug according to any one of claims 1 to 4, comprising:
a pair of earplugs;
a lower case including earplug accommodating parts for accommodating a pair of the earplugs and a pair of ear pad mounting parts, each of which has a protrusion part formed at the center to allow the user to stick ear pads thereto;
at least a pair of ear pads mounted on a pair of the ear pad mounting parts; and
an upper case which is opposed to the lower case and surrounds a pair of the earplugs and a pair of the ear pads to protect them.
